(19) Europäisches Patentamt
European Patent Office
Office européen des brevets

(11) **EP 1 169 372 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**31.10.2018 Patentblatt 2018/44**

(45) Hinweis auf die Patenterteilung:
**02.05.2003 Patentblatt 2003/18**

(21) Anmeldenummer: **00916874.1**

(22) Anmeldetag: **26.02.2000**

(51) Int Cl.:
*C08J 3/24* (2006.01)   *C08F 8/00* (2006.01)
*A61L 15/48* (2006.01)   *A61L 15/60* (2006.01)
*B01J 20/26* (2006.01)   *C08L 33/02* (2006.01)

(86) Internationale Anmeldenummer:
**PCT/EP2000/001609**

(87) Internationale Veröffentlichungsnummer:
**WO 2000/053664 (14.09.2000 Gazette 2000/37)**

(54) **PULVERFÖRMIGE, VERNETZTE, ABSORBIERENDE POLYMERE, VERFAHREN ZU IHRER HERSTELLUNG UND IHRE VERWENDUNG**

POWDERY, CROSS-LINKED ABSORBENT POLYMERS, METHOD FOR THE PRODUCTION THEREOF AND THEIR USE

POLYMERES POUDREUX, RETICULES, A POUVOIR ABSORBANT, LEUR PROCEDE DE PRODUCTION ET LEUR UTILISATION

(84) Benannte Vertragsstaaten:
**AT BE CH CY DE DK ES FI FR GB GR IE IT LI LU MC NL PT SE**

(30) Priorität: **05.03.1999 DE 19909653**

(43) Veröffentlichungstag der Anmeldung:
**09.01.2002 Patentblatt 2002/02**

(73) Patentinhaber: **Evonik Degussa GmbH**
**45128 Essen (DE)**

(72) Erfinder:
• **MERTENS, Richard**
**D-47803 Krefeld (DE)**
• **HARREN, Jörg**
**D-47807 Krefeld (DE)**

(74) Vertreter: **Herzog, Fiesser & Partner**
**Patentanwälte PartG mbB**
**Immermannstrasse 40**
**40210 Düsseldorf (DE)**

(56) Entgegenhaltungen:
EP-A- 0 233 067    EP-A- 0 574 260
DE-A- 3 503 458    DE-C- 4 020 780
US-A- 5 314 420

• **CHEMICAL ABSTRACTS, vol. 127, no. 4, 28. Juli 1997 (1997-07-28) Columbus, Ohio, US; abstract no. 51534, XP002142564 & DATABASE WPI Week 9729 Derwent Publications Ltd., London, GB; AN 1997-316703 & JP 09 124879 A (SANYO CHEM IND LTD), 13. Mai 1997 (1997-05-13)**

EP 1 169 372 B2

**Beschreibung**

Die Erfindung betrifft pulverförmige, vernetzte, Wasser, wässrige Flüssigkeiten sowie Blut absorbierende Polymere (Superabsorber), mit verbesserten Eigenschaften insbesondere mit einer verbesserten Retention und einem verbesserten Rückhaltevermögen von Flüssigkeiten unter Druck und einer verbesserten Fähigkeit Flüssigkeiten zu transportieren, deren Herstellung und deren Verwendung als Absorptionsmitttel in Hygieneartikeln und in technischen Bereichen

Superabsorber sind wasserunlösliche, vernetzte Polymere, die in der Lage sind, unter Quellung und Ausbildung von Hydrogelen große Mengen an wässrigen Flüssigkeiten und Körperflüssigkeiten, wie z. B. Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikel, wie z. B. Babywindeln, Inkontinenzprodukten oder Damenbinden.

Bei den gegenwärtig kommerziell verfügbaren Superabsorbern handelt es sich im Wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht die zunehmende Tendenz, die Sanitärartikel wie Babywindeln, Inkontinezprodukte und Damenbinden immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozeß läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozeß, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstärke zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzern und Comonomeren, wie in Patentschrift DE 196 46 484 beschrieben, vermag die Permeabilitätseigenschaften zwar zu verbessern, nicht aber auf ein Niveau, das beispielsweise den Einbau einer gegebenenfalls nur aus Superabsorbern bestehende Schicht in eine Windelkonstruktion erlaubt.

Weiterhin können Methoden zur oberflächlichen Nachvernetzung der Polymerpartikel zur Anwendung kommen. Bei der sog. Nachvernetzung werden die Carboxylgruppen der Polymermoleküle an der Oberfläche der Superabsorberpartikel mit verschiedenen Nachvernetzungsmitteln, die mit mindestens zwei der oberflächennahen Carboxylgruppen reagieren können, zur Reaktion gebracht.
Neben der Erhöhung der Gelstärke wird insbesondere die Fähigkeit zur Flüssigkeitsaufnahme unter Druck stark verbessert, da das bekannte Phänomen des Gel-Blocking unterdrückt wird, bei dem angequollene Polymerteilchen verkleben und dadurch eine weitere Flüssigkeitsaufnahme verhindert wird.

Die Oberflächenbehandlung von flüssigkeitsabsorbierenden Harzen ist bereits bekannt. Zur Verbesserung der Dispergierbarkeit wird eine ionische Komplexierung der oberflächennahen Carboxylgruppen mit polyvalenten Metallkationen in der US 4,043,952 vorgeschlagen. Die Behandlung erfolgt mit Salzen mehrwertiger Metalle, die in organischen, ggf. Wasser enthaltende Solventien, (Alkohole und andere organische Solventien), dispergiert sind.

Eine Nachbehandlung von Superabsorberpolymeren mit reaktionsfähigen, oberflächenvernetzenden Verbindungen (Alkylencarbonate) zur Erhöhung der Flüssigkeitsaufnahmefähigkeit unter Druck wird in DE-A-40 20 780 beschrieben.

Die EP 0 233 067 beschreibt wasserabsorbierende, an der Oberfläche vernetzte Harze, die durch Reaktion von einem superabsorbierenden Polymerpulver mit einer Aluminiumverbindung erhalten werden. Als Behandlungslösung findet eine Mischung aus Wasser und Diolen Verwendung, die den Einsatz von niederen Alkoholen als Lösemittel überflüssig machen soll. Es werden bevorzugt 100 Teile Vernetzerlösung auf 100 bis 300 Teile Absorber aufgebracht. Gemäß der Beispiele findet die Umsetzung mit der Aluminiumkomponente bei Raumtemperatur statt. Die dem Reaktionsmedium Wasser zugefügten Diole (z. B. Polyethylenglycol 400 und 2000, 1,3-Butandiol oder 1,5-Pentandiol) dienen dazu, ein Verklumpen des Superabsorbers bei der Behandlung mit den hier verwendeten großen Mengen an wässriger Behandlungslösung zu verhindern. Das Lösemittel wird in einer anschließenden Trocknung bei 100°C entfernt. Die so behandelten Polymere weisen ein nicht ausreichendes Eigenschaftsniveau auf, wobei eine Verbesserung der Absorptionsfähigkeit unter Druck nicht erreicht wird. Außerdem ist eine Behandlung mit großen Mengen Behandlungslösung bei modernen, kontinuierlich arbeitenden Verfahren nicht ökonomisch durchführbar.

In der WO 96/05234 wird ein Verfahren zur Herstellung superabsorbierender Polymere beschrieben, gemäß dem an der Oberfläche der mindestens 10 Gew.% Wasser enthaltenden Absorberteilchen eine vernetzte Schicht durch eine Reaktion von einem reaktiven, hydrophilen Polymeren oder einer reaktiven metallorganischen Verbindung mit einem mindestens bifunktionellen Vernetzer unter 100°C gebildet wurde. Die Polymerisate sollen ein ausgewogenes Verhältnis von Absorption, Gelfestigkeit und Permeabilität aufweisen, wobei die Meßwerte nach äußerst niedrigen Bewertungskriterien ermittelt werden. So werden beispielsweise die Absorption und die Permeabilität ohne jegliche Druckbelastung bestimmt. Nachteilig ist bei diesem bekannten-Verfahren die Verwendung von Lösemitteln und toxisch bedenklichen Vernetzungsreagentien wie z.B. den als bevorzugt genannten Polyiminen, alkoxylierten Silikon- bzw. Titan-Verbindungen und Epoxiden.

Durch eine entsprechende Behandlung von kommerziell erhältlichen Superabsorberprodukten mit Aminopolymeren in organischen Lösungsmitteln wird in WO 95/22356 und WO 97/12575 eine Verbesserung der Permeabilitäts- und Flüssigkeitstransporteigenschaften erreicht. Der gravierende Nachteil des hier beschriebenen Verfahrens liegt neben der Verwendung von toxikologisch bedenklichen Polyaminen und Polyiminen in dem Einsatz großer Mengen organischer Lösungsmittel, die für die Behandlung der Polymere notwendig sind. Der damit verbundene Sicherheitsaspekt und Kostenaufwand schließt eine großtechnische Produktion aus. Neben der toxikologischen Bedenklichkeit dieser Behandlungsmittel ist weiterhin zu berücksichtigen, daß sie unter den hohen Nachvernetzungstemperaturen auch zur Zersetzung neigen, was sich u.a. in einer Gelbfärbung der Absorberpartikel äußert.

Einen Hinweis darauf, daß unter Beibehaltung einer hohen Retentionskapazität und Aufnahmefähigkeit von Flüssigkeit unter Druck bei der Nachvernetzungsstufe ebenfalls die Permeabilitätseigenschaften drastisch gesteigert werden können, ist aus dem vorstehend beschriebenen Stand der Technik nicht zu erkennen.

Aufgabe der vorliegenden Erfindung war es daher, superabsorbierende Polymere bereitzustellen, die als Eigenschaftkombination nicht nur eine hohe Aufnahmekapazität unter Druck, sondern auch die üblicherweise gegenläufigen Eigenschaften eines hohen Retentionsvermögens und einer guten Permeabilität in sich vereinigen, d. h. ein Niveau der Eigenschaftskombination aufweisen, bei dem neben einem Retentionswert von $\geq 25$ g/g mindestens ein SFC-Wert von mindestens $30 \cdot 10^{-7}$, vorzugsweise von mindestens $50 \cdot 10^{-7}$cm$^3$ s /g vorliegt. Insbesondere lag die Aufgabe darin, superabsorbierende Polymere zur Verfügung zu stellen, die sich vor allem für die Verwendung in sehr dünnen Windelkonstruktionen mit sehr hohem Superabsorberanteil eignen. Für diesen Fall sind insbesondere Polymere mit Retentionswerten von $\geq 25$ g/g und Permeabilitätswerte von SFC $> 70 \cdot 10^{-7}$cm$^3$ s /g erforderlich.

Eine weitere Aufgabe der Erfindung war es, Herstellungsverfahren für solche superabsorbierenden Polymeren zu finden, die einfach, ökonomisch und sicher durchführbar sind, eine gleichmäßige Produktqualität liefern und bei denen insbesondere niedrige Lösungsmittelmengen verwendet und organische Lösungsmittel nach Möglichkeit vermieden werden. Darüber hinaus sollen die Verfahren ohne die Verwendung toxikologisch bedenklicher Substanzen durchführbar sein.

Die erfindungsgemäße Aufgabe wird durch die Bereitstellung eines Verfahrens zur Herstellung von pulverförmigen, an der Oberfläche nachvernetzten, Wasser, wäßrige oder seröse Flüssigkeiten sowie Blut absorbierenden Polymerisaten gelöst, wie in Anspruch 1 definiert.

Überraschenderweise ergibt sich nämlich durch die Beschichtung eines teilchenförmigen Absorberharzes mit einer wässrigen Lösung von 1,3-Dioxolan-2-on als organisches Vernetzungsmittel, das mit den oberflächennahen Molekülgruppen, vorzugsweise mit den Carboxylgruppen, in Anwesenheit eines Aluminium-Kations eines Aluminiumsalzes vorzugsweise unter Erhitzung auf 40 bis 300 °C reagiert hat, ein Superabsorberharz mit einer signifikanten Verbesserung der Permeabilitätseigenschaften bei sehr gutem Retentionsvermögen.

Völlig unerwartet führt die wässrige Lösung der erfindungsgemäßen Kombination von Nachvernetzer-Komponenten zum erwünschten Ergebnis, nämlich Superabsorberharzen mit einem hohen Retentionsvermögen auch unter Druck bei gleichzeitig ausgezeichneten Permeabilitätseigenschaften. Eine aufeinander folgende separate Anwendung sowohl einer wässrigen Lösung des organischen Nachvernetzungsmittels bzw. der wässrigen Salzlösung mit jeweiligem Erhitzen führt nicht zu einer vergleichbar guten Produktcharakteristik.

Die alleinige Verwendung von organischen Nachvernetzungsmitteln, wie beispielsweise von Alkylencarbonaten in wässriger Lösung, führt zu Produkten mit hoher Retentionskapazität, hoher Gelstärke und hohem Aufnahmevermögen unter Druck. Eine signifikante Steigerung der Permeabilität im gequollenen Zustand kann allerdings nur durch einen entsprechend höheren Grad der Vernetzung der Polymere bei der Polymerisation, bzw. einer stärkeren Nachvernetzung (erhöhte Mengen Nachvernetzungsmittel oder drastischere Bedingungen) und dem damit verbundenen Verlust an Retentionskapazität erreicht werden.

Die alleinige Nachvernetzung mit Kationen hoher positiver Ladungsdichte führt ebenfalls nicht zu Polymerisaten mit der erwünschten Eigenschaftskombination. Insbesondere lassen sich keine befriedigenden Werte bei der Flüssigkeitsaufnahme unter Druck und keine guten Permeabilitätseigenschaften erreichen. Die Behandlung von Superabsorberpolymeren nur mit mehrwertigen Kationen vermag daher nur die Geschwindigkeit der Flüssigkeitsaufnahme zu erhöhen. Eine Verbesserung der Druckstabilität oder gar der Flüssigkeitstransporteigenschaften im gequollenen Zustand wird nicht erreicht.

Erfindungsgemäß wird als organische Nachvemetzer-Komponente e) 1,3 Dioxolan-2-on eingesetzt

Die organische Nachvemetzerkomponente bzw. deren Mischungen werden in Mengen von 0,01-5 Gew.%, bevorzugt 0,1 - 2,5 Gew.% und besonders bevorzugt von 0,5 bis 1,5 Gew.%, bezogen auf das an seiner Oberfläche zu vemetzende Polymerisat, eingesetzt.

Erfindungsgemäß werden als Komponente f) wässrige Lösungen von Salzen zur Vernetzung der oberflächennahen Carboxylatgruppen eingesetzt, deren Anionen Chloride, Bromide, Sulfate, Carbonate, Nitrate, Phosphate oder organische Anionen wie Acetate und Lactate sind. Die Kationen sind Kationen von Aluminium Eingesetzt werden Aluminiumsalze und Alaune und deren unterschiedliche Hydrate wie z.B. $AlCl_3 x 6 H_2O$, $NaAl(SO_4)_2 x 12 H_2O$, $KAl(SO)_4 x 12 H_2O$ oder $Al_2(SO_4)_3 x 14$-$18 H_2O$. Besonders bevorzugt werden $Al_2(SO_4)_3$ und seine Hydrate verwendet. Eingesetzt wird die Salzkomponente, berechnet auf das Kation, in Mengen von 0,001 - 1,0 Gew.%, bevorzugt 0,005 - 0,5 Gew.%, und besonders bevorzugt 0,01 - 0,2 Gew.%, bezogen auf das Polymerisat.

Das wasserabsorbierende Polymerisat, das oberflächenvernetzt wird, wird durch Polymerisation von a) 55-99,9 Gew% eines einfach ungesättigten Monomeren mit Säuregruppen erhalten. Hierbei sind carboxylgruppenhaltige Monomere bevorzugt, wie z.B. Acrylsäure, Methacrylsäure oder 2-Acrylamido-2-methylpropansulfonsäure oder Mischungen dieser Monomeren.. Es ist bevorzugt, daß mindestens 50% und besonders bevorzugt mindestens 75% der Säuregruppen Carboxyl-Gruppen sind. Die Säuregruppen sind zu mindestens zu 25 Mol% neutralisiert , d.h. liegen als Natrium-, Kalium- oder Ammoniumsalze vor. Bevorzugt liegt der Neutralisationsgrad bei mindestens 50 mol%. Besonders bevorzugt ist ein Polymerisat, das durch Polymerisation von Acrylsäure oder Methacrylsäure, deren Carboxylgruppen zu 50-80 Mol% neutralisiert ist, in Gegenwart von Vernetzern erhalten wurde.

Als weitere Monomere b) können für die Herstellung der absorbierenden Polymerisate 0-40 Gew% ethylenisch ungesättigte mit a) copolymerisierbarer Monomere, wie z. B. Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Über 40 Gew% dieser Monomerern können die Quellfähigkeit der Polymerisate verschlechtern.

Als Vernetzerkomponente c), die während der Polymerisation von a) und b) vorhanden ist, können alle Verbindungen verwendet werden, die mindestens zwei ethylenisch ungesättigte Doppelbindungen oder eine ethylenisch ungesättigte Doppelbindung und eine gegenüber Säuregruppen der Monomeren a) reaktive funktionelle Gruppe oder mehrere gegenüber Säuregruppen reaktive funktionelle Gruppen tragen. Beispielhaft seien genannt: aliphatische Amide wie z. B. das Methylenbisacryl- bzw. -methacrylamid oder Ethylenbisacrylamid, ferner aliphatische Ester von Polyolen oder alkoxylierten Polyolen mit ethylenisch ungesättigten Säuren, wie Di(meth)acrylate oder Tri(meth)acrylate, Butandiol- oder Ethylenglykol, Polyglykolen, Trimethylolpropan, Di- und Triacrylatester des, vorzugsweise mit 1 bis 30 Mol Alkylenoxid oxalkylierten, vorzugsweise ethoxylierten Trimethylolpropans, Acrylat- und Methacrylatester von Glycerin und Pentaerythrit, sowie des mit vorzugsweise 1 bis 30 Mol Ethylenoxid oxethylierten Glycerins und Pentaerythrits, femer Allylverbindungen wie Allyl(meth)acrylat, alkoxyliertes Allyl(meth)acrylat mit vorzugsweise 1 bis 30 Mol Ethylenoxid umgesetzt, Triallylcyanurat, Triallylisocyanurat, Maleinsäurediallylester, Polyallylester, Tetraallyloxiethan, Triallylamin, Tetraallylethylendiamin, Allylester der Phosphorsäure bzw. phosphorigen Säure, femer vernetzungsfähige Monomere, wie N-Methylolverbindungen von ungesättigten Amiden wie von Methacrylamid oder Acrylamid und die davon abgeleiteten Ether. Mischungen der genannten Vernetzer können ebenfalls eingesetzt werden. Der Anteil an den vernetzenden Comonomeren liegt bei 0,1 bis 5 Gew%, bevorzugt bei 0,01 bis 3,0 Gew%, bezogen auf die Gesamtmenge der Monomeren.

Als wasserlösliche Polymere d) können in den erfindungsgemäßen absorbierenden Polymerisaten 0-30 Gew.% wasserlösliche Polymerisate, wie teil- oder vollverseifter Polyvinylalkohol, Polyvinylpyrrolidon, Stärke oder Stärkederivate, Polyglykole oder Polyacrylsäuren enthalten, vorzugsweise einpolymerisiert sein. Das Molekulargewicht dieser Polymeren ist unkritisch, solange sie wasserlöslich sind. Bevorzugte wasserlösliche Polymere sind Stärke und Polyvinylalkohol. Der bevorzugte Gehalt an solchen wasserlöslichen Polymeren im erfindungsgemäß absorbierenden Polymerisat liegt bei 0-30 Gew.%, vorzugsweise 0-5 Gew%, bezogen auf die Gesamtmenge der Komponenten a) bis d). Die wasserlöslichen Polymere, vorzugsweise synthetische wie Polyvinylalkohol, können auch als Pfropfgrundlage für die zu polymerisierenden Monomeren dienen.

Zur Initiierung der radikalischen Polymerisation werden die gebräuchlichen Initiatoren wie z.B. Azo- oder Peroxoverbindungen, Redoxsysteme oder UV-Initiatoren (Sensibilisatoren) verwendet.

Die Herstellung der erfindungsgemäßen Polymerisate erfolgt vorzugsweise nach zwei Methoden:

Nach der ersten Methode wird das teilneutralisierte Monomere a), vorzugsweise die Acrylsäure in wäßriger Lösung in Gegenwart von Vernetzern und ggf. weiterer Komponenten durch radikalische Polymerisation in ein Gel überführt, das zerkleinert, getrocknet, gemahlen und auf die gewünschte Partikelgröße abgesiebt wird. Diese Lösungspolymerisation kann kontinuierlich oder diskontinuierlich durchgeführt werden. Der Stand der Technik weist ein breites Spektrum an Variationsmöglichkeiten hinsichtlich der Konzentrationsverhältnisse, Temperaturen, Art und Menge der Initiatoren aus. Typische Verfahren sind in den folgenden Veröffentlichungen beschrieben: US 4 286 082, DE 27 06 135 und US 4 076 663

Auch die inverse Suspensions- und Emulsionspolymerisation kann zur Herstellung der erfindungsgemäßen Produkte angewendet werden. Gemäß diesen Prozessen wird eine wäßrige, teilneutralisierte Lösung der Monomeren a), vorzugsweise Acrylsäure mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren d) als Pfropfgrundlage über die Monomerlösung oder durch direkte Vorlage in die Ölphase. Anschließend wird das Wasser azeotrop aus dem Gemisch entfernt und das Polymerisat abfiltriert und ggf. getrocknet. Die Vernetzung kann durch Einpolymerisation eines in der Monomerenlösung gelösten polyfunktionellen Vernetzers und/oder durch Reaktion geeigneter Vernetzungsmittel mit funktionellen Gruppen des Polymeren während der Polymerisationsschritte erfolgen. Die Verfahren sind beispielsweise in den Veröffentlichungen US 43 40 706, DE 3713 601, DE 28 40 010 und WO 96/05234 beschrieben

Die Trocknung des Polymerisatgels erfolgt bis zu einem Wassergehalt von 0,5-25 Gew.%, vorzugsweise von 1 bis 10 Gew.%, besonders bevorzugt 1 bis 8 Gew.% bei Temperaturen, die üblicherweise im Bereich von 100 - 200 °C liegen.

Hinsichtlich der Teilchenform des erfindungsgemäßen absorbierenden Polymerisaten gibt es keine besonderen Einschränkungen. Das Polymerisat kann in Form von Kügelchen vorliegen, die durch inverse Suspensionspolymerisation erhalten wurden, oder in Form von unregelmäßig geformten Teilchen, die durch Trocknung und Pulverisierung der Gelmasse aus der Lösungspolymerisation stammen. Die Teilchengröße liegt normalerweise unter 3000 μm, bevorzugt zwischen 20 und 2000 μm, und besonders bevorzugt zwischen 150 und 850 μm.

Die erfindungsgemäßen Nachvernetzerkomponenten werden in Form ihrer wässrigen Lösungen aufgebracht. Geeignete Lösungsmittel sind Wasser und ggf. polare, mit Wasser mischbare organische Lösungsmittel wie beispielsweise Aceton, Methanol, Ethanol oder 2-Propanol bzw. deren Gemische. Der Begriff wäßrige Lösung im Sinne der Erfindung bedeutet in Bezug auf die Lösungsmittelkomponente, daß neben dem Wasser auch noch andere organische Lösungsmittel enthalten sein können. Die Konzentration der jeweiligen Nachvernetzerkomponente in dem wässrigen Lösungsmittel kann in weiten Grenzen schwanken und liegt im Bereich von 1 bis 80 Gew.%, vorzugsweise im Bereich von 5 bis 65 Gew.% und ganz besonders bevorzugt in einem Bereich von 10 bis 40 Gew.%. Das bevorzugte Lösungsmittel für das organische Nachvernetzungsmittel bzw. die Salzkomponente ist Wasser, das in einer Menge von 0,5 - 10 Gew.%, bevorzugt 0,75 - 5 Gew.% und besonders bevorzugt 1,0 - 4 Gew.%, bezogen auf das Polymerisat verwendet wird.

Sofern der organische Nachvernetzer und die Salzkomponente in einer wässrigen Lösung vorliegen, können die lösbaren Mengen beider Komponeten durch Aussalzeffekte begrenzt sein und sind entsprechend der Zusammensetzung anzupassen. Da aus sicherheitstechnischen Gründen zur Vermeidung von Explosionen die Menge an organischem Solvens so gering wie möglich gehalten werden soll, ist eine stabile Mischphase Wasser/organisches Lösungs-mittel/organische Nachvernetzerverbindung/Salzkomponete nicht über beliebige Konzentrationen der Verbindung zu erreichen. Eine bevorzugte Lösung besteht beispielsweise aus 1,5 - 3 Gew.Teilen Wasser, 0,5 - 1 Gew.Teilen organische Nachvernetzerverbindung und 0,4 - 0,6 Gew.Teilen eines anorganischen Salzes. Die gesamte Menge an Lösungsmittel wird üblicherweise im Bereich von 0,5-12 Gew.%, bevorzugt bei 1 - 7 Gew.% und besonders bevorzugt bei 1 - 5 Gew.% bezogen auf das Polymerisat eingesetzt.

Um die Flüssigkeitsmengen, die auf das Polymerisat aufgebracht werden, zu reduzieren, können neben Wasser und den oben genannten organischen Solventien auch andere Lösungsvermittler zum Einsatz kommen, wie zum Beispiel anorganische oder organische Säuren oder Komplexbildner.

Abhängig von der Löslichkeit der beiden Komponenten e) und f) wird die Lösung vor dem Aufbringen auf das Polymerisat auf 20-100 °C, bevorzugt auf 20-60 °C erwärmt. Ein getrenntes, aber gleichzeitiges Zudosieren von einer Lösung des organischen Nachvernetzers und einer Lösung der Salzkomponente ist ebenfalls möglich, wenn eine homogene Verteilung beider Komponenten auf dem Polymerisat gewährleistet ist und das Material anschließend thermisch nachbehandelt wird. Bevorzugt ist das Aufbringen einer einzigen Lösung auf das Polymerisat, in der beide Komponenten gelöst sind.

Die Nachvernetzerlösung sollte sehr gut mit den Polymerteilchen vermischt werden. Geeignete Mischaggregate zum Aufbringen der Nachvernetzerlösung sind z.B. Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymerisat-Pulver mittels rotierender Messer in schneller Frequenz gemischt wird (Schugi-Mischer). Es besteht auch die Möglichkeit, die Beschichtung des Polymerisates während eines Verfahrensschrittes bei der Herstellung des Polymerisates vorzunehmen. Hierzu ist besonders der Prozeß der inversen Suspensionspolymerisation geeignet.

Nachdem die Nachvernetzerlösung mit den Polymerteilchen vermischt worden ist, erfolgt die Nachvernetzungsreaktion vorzugsweise bei Temperaturen im Bereich von 40°C bis 300°C, bevorzugt von 80°C bis 250°C und besonders bevorzugt von 160°C bis 210°C. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drehrohröfen, Wirbelbetttrockner, Tellertrockner, Paddeltrockner oder Infrarottrockner genannt.

Die Polymeren gemäß der Erfindung können in großtechnischer Weise nach bekannten kontinuierlich oder diskontinuierlich hergestellt werden.

Die erfindungsgemäßen Polymerisate können für weite Anwendungsgebiete eingesetzt werden. Wenn sie z.B. als Absorbierungsmittel in Damenbinden, Windeln oder in Wundabdeckungen verwendet werden, besitzen sie die Eigenschaft, dass sie große Mengen an Menstruationsblut, Urin oder anderen Körperflüssigkeiten schnell absorbieren. Da die erfindungsgemäßen Mittel die absorbierten Flüssigkeiten auch unter Druck zurückhalten und zusätzlich in der Lage sind, im gequollenen Zustand weitere Flüssigkeiten innerhalb der Konstruktion zu verteilen, werden sie besonders bevorzugt in höheren Konzentrationen, in Bezug auf das hydrophile Fasermaterial wie z.B. Fluff eingesetzt als dies bisher möglich war. Sie eignen sich auch für den Einsatz als homogene Superabsorberschicht ohne Fluffanteil innerhalb der Windelkonstruktion, wodurch besonders dünne Windeln möglich sind. Weiterhin eignen sich die Polymere zum Einsatz in Hygieneartikel (Inkontinenzprodukte) für Erwachsene.

Die erfindungsgemäßen Polymeren werden auch in Absorberartikeln eingesetzt, die für die verschiedensten Verwendungen geeignet sind, so z.B. durch Mischen mit Papier oder Fluff oder synthetischen Fasern oder durch Verteilen der Superabsorber zwischen Substraten aus Papier, Fluff oder nicht gewebten Textilien oder durch Verarbeitung in Trägermaterialien zu einer Bahn. Des Weiteren finden die erfindungsgemäßen Polymeren auch überall dort Verwendung, wo wässrige Flüssigkeiten absorbiert werden müssen, wie z.B. bei Kabelummantelungen, bei Konstruktionen zur Aufnahme von Körperflüssigkeiten, in geschäumten und nicht geschäumten Flächengebilden, in Verpackungsmaterialien, Lebensmittelverpackungen, im Agrarbereich bei der Pflanzenaufzucht, Konstruktionen für die Pflanzenaufzucht, als Bodenverbesserungsmittel und als Wasserspeicher sowie als Wirkstoffträger mit einer zeitlich verzögerten Freisetzung des Wirkstoffes in die Umgebung.

Die erfindungsgemäßen Superabsorber zeigen überraschenderweise eine bedeutende Verbesserung der Permeabilität, d.h. eine Verbesserung des Flüssigkeitstransportes im gequollenen Zustand. Es werden Polymerisate mit Permeabilitäts-Werten (SFC) von bis zu $70 \cdot 10^{-7}$ cm$^3$ s/g bei einer Retention (TB) von mindestens 27 g/g erhalten, vorzugsweise Polymere mit SFC-Werten von $> 70 \cdot 10^{-7}$ bis $\geq 150 \cdot 10^{-7}$ cm$^3$ s/g bei einer Retention (TB) von mindestens 25 g/g. Neben diesen ausgezeichneten SFC- und Retentionswerten zeigen die erfindungsgemäßen Polymere Messwerte für die Flüssigkeitsaufnahme unter Druck (AAP 0,7) von mindestens 18 g/g.

Die erfindungsgemäßen Produkte mit dieser hervorragenden Eigenschaftskombination aus sehr hohen SFC-Werten, hoher Retention und hoher Absorption unter Druck können ohne die Verwendung toxikologisch bedenklicher Substanzen hergestellt werden.

Wie aus den folgenden Beispielen zu entnehmen ist, ist die erfindungsgemäße Nachvernetzung auf eine Vielzahl chemisch verschieden aufgebauter absorbierender Polymerisate anwendbar. Damit entfällt die Notwendigkeit, bereits während der Herstellung der Polymerisate auf spezielle Vernetzerkombinationen, Comonomere oder aufwendige Nachbehandlungsverfahren zurückgreifen zu müssen, um eine auch nur etwas erhöhte Permeabilität zu erreichen.

**Testmethoden**

Zur Charakterisierung der erfindungsgemäßen, absorbierenden Polymerisate werden Retention (TB), Aufnahme unter Druck (AAP) und die Durchlässigkeit für 0,9%ige Kochsalzlösung im gequollenen Zustand (SFC) bestimmt.

a) Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat läßt man als Blindwert mitlaufen. Retention = Auswaage-Blindwert/Einwaage [g/g]

b) Flüssigkeitsaufnahme unter Druck (AAP-Test, gemäß EP 0 339 461)
Die Aufnahme unter Druck (Druckbelastung 50 g/cm$^2$) wird nach einer in der EP 0339461, Seite 7, beschriebenen Methode bestimmt. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor gewogene Zylinder wird anschließend auf eine Glasfilterplatte gestellt, die sich in einer Schale mit 0,9%iger NaCl-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

AAP = Auswaage (Zylindereinheit + Superabsorber)-Einwaage (Zylindereinheit + vollgesogener Superabsorber) / Einwaage Superabsorber

c) Permeabilität im gequollenen Zustand (SFC-Test, gemäß WO 95/22356) In einen Zylinder mit Siebboden werden ca. 0,9 g Superabsorbermaterial eingewogen und sorgfältig auf der Siebfläche verteilt. Das Superabsorbermaterial läßt man in JAYCO synthetischen Urin [Zusammensetzung: 2,0 g Kaliumchlorid; 2,0 g Natriumsulfat; 0,85 g Ammoniumdihydrogenphosphat; 0,15 g Ammoniumhydrogenphosphat; 0,19 g Calciumchlorid; 0,23 g Magnesiumchlorid als wasserfreie Salze in 11 destilliertem Wasser gelöst] 1 Stunde lang gegen einen Druck von 20 g/cm$^2$ quellen. Nach Erfassung der Quellhöhe des Superabsorbers läßt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Meßtemperatur 20-25°C) während der Messung bezüglich der Gelbettbeschaffenheit gewährleistet. Der auf den gequollenen Superabsorber wirkende Druck ist weiterhin 20 g/cm$^2$. Mit Hilfe eines Computers und einer Waage wird die Flüssigkeitsmenge, die die Gelschicht als Funktion der Zeit passiert in Intervallen von 20 Sekunden innerhalb einer Zeitperiode von 10 Minuten erfaßt. Die Fließrate g/s durch die gequollene Gelschicht wird mittels Regressionsanalyse mit Extrapolation der Steigung und Ermittlung des Mittelpunkts auf den Zeitpunkt t=0 der Fließmenge innerhalb der Minuten 2-10 ermittelt. Die Berechnung des SFC-Wertes (K) berechnet sich wie folgt:

$$K= \frac{F_s(t=0)\cdot L_0}{r\cdot A\cdot \Delta P} = \frac{F_s(t=0)\cdot L_0}{139506}$$

Wobei:

$F_s(t = 0)$     die Fließrate in g/s
$L_0$     die Dicke der Gelschicht in cm
r     die Dichte der NaCl-Lösung (1,003 g/cm$^3$)
A     die Fläche der Oberseite der Gelschicht im Meßzylinder (28,27 cm$^2$)
$\Delta P$     der hydrostatische Druck, der auf der Gelschicht lastet (4920 dyne/cm$^2$)
und K     der SFC-Wert ist [cm$^3$*s*g$^{-1}$]

Die formale Addition der Zahlenwerte der Teebeutelretention und des SFC-Wertes verdeutlicht den sprunghaften Anstieg dieser Eigenschaftskombination bei den erfindungsgemäßen Polymerisaten im Vergleich zu unbehandeltem Superabsorberpulver oder Produkten die nach bekannten Methoden oberflächlich nachvernetzt wurden. Der Zahlenwert wird bei den erfindungsgemäßen Produkten nicht durch einen hohen Beitrag einer der beiden Werte erreicht (z. B. eines hohen TB-Retentionswertes und eines niedrigen SFC-Wertes und umgekehrt).

**Beispiele**

In den Beispielen und Vergleichsbeispielen wurde das zur nachvernetzenden Oberflächenbehandlung jeweils eingesetzte Pulver auf eine Teilchengröße von 150 $\mu$m bis 850 um abgesiebt.

**Beispiel 1**

Eine durch Lösungspolymerisation erhaltene, mit 0,7 Gew.% bezogen auf Acrylsäure, Polyethylenglykoldiacrylat vernetzte Polyacrylsäure, die bis 70 Mol-% neutralisiert als Natriumsalz vorlag, wurde nach dem Trocknen und Mahlen auf ein Pulver mit einer Teilchengröße von 150-850 $\mu$m abgesiebt (Pulver A). 100 g Pulver A wurden unter kräftigem Rühren mit einer Lösung aus 1 g 1,3-Dioxolan-2-on, 3 g Wasser und 0,5 g Aluminiumsulfat-18-Hydrat vermischt und anschließend für 30 min. in einem Ofen, der auf 180 °C temperiert war, erhitzt.
Zum Vergleich wurden 100 g Pulver A mit einer Lösung aus 1 g 1,3-Dioxolan-2-on und 3 g Wasser vermischt und anschließend für 30 min. in einem Ofen, der auf 180 °C temperiert war, erhitzt (Vergteichsbeispiel 1, entsprechend DE 4020780). Zum weiteren Vergleich wurden 100 g Pulver A mit einer Lösung aus 3 g Wasser und 0,5 g Aluminiumsulfat-18-Hydrat vermischt und anschließend für 30 min. in einem Ofen, der auf 180°C temperiert war, erhitzt (Vergleichsbeispiel 2).

| Produkt | TB [g/g] | AAP$_{0.7}$ [g/g] | SFC [$10^{-7}$cm$^3$s/g] | TB+SFC |
|---|---|---|---|---|
| Pulver A | 33,5 | 8,5 | 0 | 33,5 |
| Beispiel 1 | 29,0 | 23,5 | 70 | 99 |
| Vergleichsbeispiel 1 | 29,0 | 24,5 | 15 | 43 |
| Vergleichsbeispiel 2 | 32,5 | 11 | 0 | 32,5 |

**Beispiele 2-6**

Fünf pulverförmige, mit unterschiedlichen Mengen an Polyethylenglykoldiacrylat radikalisch polymerisierte Polyacrylsäuren (Pulver B, C, D, E und F, je 100 g), die zu 70 Mol-% als Natriumsalz vorlagen, wurden nach dem Trocknen und Mahlen auf 150-850 $\mu$m abgesiebt und unter kräftigem Rühren mit einer Lösung aus 1 g 1,3-Dioxolan-2-on, 3 g Wasser und 0,5 g Aluminiumsulfat-18-Hydrat vermischt und anschließend für 30 min. in einem Ofen, der auf 180 °C temperiert war, erhitzt.

| Produkt | TB [g/g] | Vernetzer Gew.%/AcS* | AAP$_{0.7}$ [g/g] | SFC [$10^{-7}$cm$^3$s/g] | TB+SFC |
|---|---|---|---|---|---|
| Pulver B | 30 | 1,1 | 12 | 0 | 30 |
| Beispiel 2 | 26,5 | | 22,5 | 110 | 136,5 |
| Pulver C | 32,5 | 0,8 | 10 | 0 | 32,5 |
| Beispiel 3 | 29 | | 23,5 | 65 | 94 |
| Pulver D | 35 | 0,6 | 11,5 | 0 | 35 |
| Beispiel 4 | 30 | | 23 | 55 | 78 |
| Pulver E | 29 | 1,15 | 12 | 0 | 29 |
| Beispiel 5 | 25,5 | | 24 | 150 | 179 |
| Pulver F | 38 | 0,45 | 9 | 0 | 38 |
| Beispiel 6 | 31 | | 24,5 | 45 | 76 |
| *: Acrylsäure | | | | | |

**Beispiele 7-11**

5 vernetzte Polyacrylsäuren (Pulver E-I) wurden durch einen Herstellungsprozeß gewonnen, bei dem der Gehalt an Acrylsäure, die zu 70% neutralisiert war (AcS) in der wäßrigen Monomerenlösung variiert wurde (22-30 Gew.%, siehe Tabelle) und mit 0,7 Gew.%, bezogen auf Acrylsäure, einer Mischung aus zwei Vernetzern Triallylamin und Polyethylenglykoldiacrylat vernetzt wurde. Nach dem Trocknen und Mahlen des Polymerisates wurde auf 150-850 $\mu$m Teilchengröße abgesiebt und je 100 g der Pulver

a) unter kräftigem Rühren mit einer Lösung aus 1 g 1,3-Dioxolan-2-on, 2,5 g Wasser und 0,5 g Aluminiumsulfat-14-Hydrat vermischt und anschließend für 60 min. in einem Ofen, der auf 175 °C temperiert war, erhitzt (Beispiele 7-11), bzw.
b) unter kräftigem Rühren mit einer Lösung aus 1 g 1,3-Dioxolan-2-on und 3 g Wasser vermischt und anschließend für 60 min. in einem Ofen, der auf 175 °C temperiert war, erhitzt (Vergleichsbeispiele 3-7)

| Produkt | Gew.% AcS | TB [g/g] | AAP$_{0.7}$ [g/g] | SFC [$10^{-7}$cm$^3$s/g] | TB+SFC |
|---|---|---|---|---|---|
| Pulver E | 30 | 36,5 | | | |
| Beispiel 7 | | 30 | 23,5 | 40 | 70 |
| Vergleichsbeispiel 3 | | 30 | 24 | 22 | 52 |
| Pulver F | 28 | 36 | | | |
| Beispiel 8 | | 29,5 | 24,5 | 52 | 81,5 |
| Vergleichsbeispiel 4 | | 30 | 25,5 | 25 | 55 |
| Pulver G | 26 | 35,5 | | | |
| Beispiel 9 | | 29 | 25 | 62 | 91 |
| Vergleichsbeispiel 5 | | 30 | 25 | 15 | 45 |
| Pulver H | 24 | 36 | | | |
| Beispiel 10 | | 29,5 | 24 | 60 | 89,5 |

(fortgesetzt)

| Produkt | Gew.% AcS | TB [g/g] | AAP$_{0.7}$ [g/g] | SFC [$10^{-7}$cm$^3$s/g] | TB+SFC |
|---|---|---|---|---|---|
| **Vergleichsbeispiel 6** | | 30 | 24,5 | 25 | 55 |
| **Pulver I** | 22 | 35 | | | |
| **Beispiel 11** | | 29,5 | 25,5 | 88 | 117,5 |
| **Vergleichsbeispiel 7** | | 30 | 25,5 | 24 | 54 |

**Vergleichbeispiele 8 - 13**

20 g der Polymerpulver (I) bzw. (M), Favor® SXM 6860 (siehe Vergleichsbeispiel 14) werden mit den nachfolgenden Lösungen/Dispersionen für 1 h auf 68 °C erhitzt. Nach dem Abkühlen wird das Polymer abfiltriert und 1 h bei 80 °C im Trockenschrank getrocknet (vgl. US 4043952).

Pulver (1) +

a) 0,7 g Zinkacetat in 60 g Methanol/Wasser (90/10) (Vergleichsbeispiel 8).
b) 0,18 g basisches Aluminiumacetat (Aluminiumhydroxidacetat) in 60 g Methanol (Vergleichsbeispiel 9).
c) 0,1 g Al$_2$(SO$_4$)$_3$ x 14H$_2$O in 60 g Methanol/Wasser (90/10) (Vergleichsbeispiel 10).

Pulver (M)(Favor® SXM 6860)+
a) 0,7 g Zinkacetat in 60 g Methanol/Wasser (90/10) (Vergleichsbeispiel 11)
b) 0,18 g basisches Aluminiumacetat (Aluminiumhydroxidacetat) in 60 g Methanol (Vergleichsbeispiel 12).
c) 0,1 g Al$_2$(SO$_4$)$_3$ x 14H$_2$O in 60 g Methanol/Wasser (90/10) (Vergleichsbeispiel 13).

| Produkt | TB [g/g] | AAP$_{0.7}$ [g/g] | SFC [$10^{-7}$cm$^3$s/g] | TB+SFC |
|---|---|---|---|---|
| **Vergleichsbeispiel 8** | 31,5 | 8 | 0 | 31,5 |
| **Vergleichsbeispiel 9** | 32 | 8 | 0 | 32 |
| **Vergleichsbeispiel 10** | 31 | 9 | 0 | 31 |
| **Vergleichsbeispiel 11** | 30,5 | 22 | 4 | 34,5 |
| **Vergleichsbeispiel 12** | 31 | 22 | 5 | 36 |
| **Vergleichsbeispiel 13** | 31 | 22 | 5 | 36 |
| **Beispiel 9** | 29 | 25 | 62 | 91 |

**Vergleichbeispiel 14**

100g Favor® SXM 6860 (Handelsprodukt der Firma Stockhausen GmbH & Co., oberflächen nachvernetztes Polyacrylat) wird unter kräftigem Rühren mit einer Lösung aus 2,5 g Wasser und 0,5 g Aluminiumsulfat-14-Hydrat vermischt und anschließend für 30 min. in einem Ofen, der auf 180 °C temperiert war, erhitzt.

| | TB [g/g] | AAP$_{0.7}$ [g/g] | SFC [$10^{-7}$cm$^3$s/g] | TB+SFC |
|---|---|---|---|---|
| **Pulver M** | 31,5 | 25,5 | 5 | 36,5 |
| **Vergleichsbeispiel 14** | 27 | 21,5 | 15 | 42 |

**Beispiele 12-13**

Je 100 g pulverförmige native Wachsmaisstärke (Cerestar SS 4201) bzw.
Polyvinylalkohol (Mowiol® 5/88) enthaltende Polyacrylsäure, die zu 70 Mol% neutralisiert vorlag (Pulver N, 5% Stärke und Pulver O, 3,5 % PVA), wurden a) unter kräftigem Rühren mit einer Lösung aus 1 g 1,3-Dioxolan-2-on, 2,5 g Wasser und 0,5 g Aluminiumsulfat-14-Hydrat vermischt und anschließend für 90 min. in einem Ofen, der auf 170 °C temperiert war, erhitzt (Beispiele 12 und 13), bzw.
b) unter kräftigem Rühren mit einer Lösung aus 1 g 1,3-Dioxolan-2-on und 3 g Wasser vermischt und anschließend für 30 min. in einem Ofen, der auf 180 °C temperiert war, erhitzt (Vergleichsbeispiele 15 und 16)

| Produkt | TB [g/g] | AAP$_{0.7}$ [g/g] | SFC [$10^{-7}$cm$^3$s/g] | TB+SFC |
|---|---|---|---|---|
| Pulver N | 28 | | | |
| Beispiel 12 | 24 | 22,5 | 115 | 139 |
| Vergleichsbeispiel 15 | 24 | 23 | 38 | 62 |
| Pulver O | 36 | | | |
| Beispiel 13 | 30 | 23 | 51 | 81 |
| Vergleichsbeispiel 16 | 30 | 24,5 | 20 | 50 |

**Beispiel 14**

100 g eines vemetzten, pulverförmigen Copolymers aus Acrylsäure und 2 Gew.% eines Methoxypolyethylenglycolmonomethacrylsäureesters (17 EO) (Pulver P), bei dem die Acrylsäure zu 70 Mol% als Natriumsalz vorlag, wurde unter kräftigem Rühren mit einer Lösung aus 1 g 1,3-Dioxolan-2-on, 2,5 g Wasser und 0,5 g Aluminiumsulfat-14-Hydrat vermischt und anschließend für 60 min. in einem Ofen, der auf 175 °C temperiert war, erhitzt.
Zum Vergleich wurden 100 g Pulver mit einer Lösung aus 1 g 1,3-Dioxolan-2-on und 3 g Wasser vermischt und anschließend für 60 min. in einem Ofen, der auf 175 °C temperiert war, erhitzt (Vergleichsbeispiel 17)

| Produkt | TB [g/g] | AAP$_{0.7}$ [g/g] | SFC [$10^{-7}$cm$^3$s/g] | TB+SFC |
|---|---|---|---|---|
| Pulver P | 35 | | | |
| Beispiel 14 | 28,5 | 24 | 100 | 128,5 |
| Vergleichsbeisplel 17 | 28 | 25 | 25 | 53 |

**Beispiel 15-17**

Durch Lösungspolymerisation gewonnene, radikalisch polymerisierte Polyacrylsäure (Gehalt an Polyethylenglykoldiacrylat als bifunktionellem Vernetzer: 0,8 Gew.%, 0,7 Gew.% bzw. 1,1 Gew.%), die bis 70 Mol-% als Natriumsalz neutralisiert vorlag, wurde nach dem Trocknen und Mahlen auf 150-850 $\mu$m abgesiebt (Pulver C, Pulver Q bzw. Pulver S). Das jeweilige Pulver wird kontinuierlich mit 80 kg/h einem Paddelmischer zugeführt und mit 4 Gew.-% einer Lösung aus 1,3-Dioxolan-2-on, Wasser, und Aluminiumsulfat-18-Hydrat (1 Teil/ 2,5 Teile/ 0,5 Teile) vermischt. Die Behandlungslösung wird mittels einer Zweistoffdüse im Mischer fein verteilt aufgegeben.
Zur thermischen Behandlung werden kontinuierlich 80 kg/h der Mischung in einen Trockner dosiert, der mit rotierenden, diskusförmigen Mischelementen ausgerüstet ist, die mit Dampf auf 186 °C beheizt werden. Anschließend wird die Mischung im Wirbelbett mit Luft abgekühlt.

| Produkt | TB [g/g] | AAP$_{0.7}$ [g/g] | SFC [$10^{-7}$cm$^3$s/g] | TB+SFC |
|---|---|---|---|---|
| Pulver C | 32,5 | 10 | 0 | 32,5 |
| Beispiel 15 | 28,0 | 24,0 | 112 | 140 |
| Beispiel 16 | 23,5 | 21,5 | 188 | 211,5 |
| Beispiel 17 | 30 | 23,5 | 55 | 85 |

**Beispiel 18**

100 g einer pulverförmigen, vernetzten Polyacrylsäure (Pulver C), die bis 70 Mol-% als Natriumsalz neutralisiert vorlag, wurde nach dem Trocknen und Mahlen auf 150-850 $\mu$m abgesiebt und unter kräftigem Rühren mit einer Lösung aus 1 g 1,3-Dioxolan-2-on, 3 g Wasser und 0,7 g Aluminiumchlorid-6-Hydrat vermischt und anschließend für 30 min. in einem Ofen, der auf 180 °C temperiert war, erhitzt.

| Produkt | TB [g/g] | AAP$_{0.7}$ [g/g] | SFC [$10^{-7}$cm$^3$s/g] | TB+SFC |
|---|---|---|---|---|
| Pulver C | 32,5 | 10 | 0 | 32,5 |
| Beispiel 18 | 29,5 | 23,0 | 62 | 91,5 |

**Beispiel 19** (nicht erfindungsgemäß)

100 g einer pulverförmigen, vernetzten Polyacrylsäure (Pulver C), die bis 70 Mol-% als Natriumsalz neutralisiert vorlag, wurde nach dem Trocknen und Mahlen auf 150-850 $\mu$m abgesiebt und unter kräftigem Rühren mit einer Lösung aus 1 g 1,3-Dioxolan-2-on, 3 g Wasser und 0,7 g Eisen(III)chlorid-6-Hydrat vermischt und anschließend für 30 min. in einem Ofen, der auf 180 °C temperiert war, erhitzt.

|  | TB [g/g] | AAP$_{0.7}$ [g/g] | SFC [$10^{-7}$cm$^3$s/g] | TB+SFC |
|---|---|---|---|---|
| **Pulver C** | 32,5 | 10 | 0 | 32,5 |
| **Beispiel 19** | 29 | 22,5 | 46 | 75 |

**Beispiele 20 und 21** (nicht erfindungsgemäß)

100 g einer pulverförmigen, vernetzten Polyacrylsäure (Pulver T), die bis 70 Mol-% als Natriumsalz neutralisiert vorlag, wurde nach dem Trocknen und Mahlen auf 150-850 $\mu$m abgesiebt und unter kräftigem Rühren mit einer Lösung aus 1 g 1,3-Dioxolan-2-on, 3 g Wasser und 0,1 g Calziumacetat-Hydrat (Beispiel 20), bzw. Magnesiumacetat-4-Hydrat (Beispiel 21) vermischt und anschließend für 30 min. in einem Ofen, der auf 185 °C temperiert war, erhitzt.

|  | TB [g/g] | AAP$_{0.7}$ [g/g] | SFC [$10^{-7}$cm$^3$s/g] | TB+SFC |
|---|---|---|---|---|
| **Pulver T** | 31,5 | 10 | 0 | 31,5 |
| **Beispiel 20** | 28,5 | 23,5 | 40 | 68,5 |
| **Beispiel 21** | 29,0 | 24 | 42 | 71 |

**Beispiele 22-27**

Zwei pulverförmige, unterschiedlich stark vernetzte Polyacrylsäuren (Pulver A bzw. C, 100 g), die bis 70 Mol-% als Natriumsalz neutralisiert vorlagen, wurden nach dem Trocknen und Mahlen auf 150-850 $\mu$m abgesiebt und unter kräftigem Rühren mit einer Lösung aus den in der Tabelle angegebenen Mengen 1,3-Dioxolan-2-on, Wasser und Aluminiumsulfat-18-Hydrat vermischt und anschließend für in einem Ofen bei der angegebenen Temperatur und Zeitdauer erhitzt.

| Produkt | Al$_2$(SO$_4$)$_3$** [g] | EC* [g] | H$_2$O [g] | TB [g/g] | AAP$_{0.7}$ [g/g] | SFC [$10^{-7}$cm$^3$s/g] | TB+SFC | T/t [°C/min] |
|---|---|---|---|---|---|---|---|---|
| **Pulver A** |  |  |  | 33,5 | 9 | 0 | 33,5 |  |
| **Beispiel 22** | 0,4 | 1 | 2,25 | 29 | 23 | 62 | 91 | 175/60 |
| **Beispiel 23** | 0,45 | 0,75 | 2,5 | 29 | 22,5 | 65 | 94 | 180/40 |
| **Beispiel 24** | 0,75 | 0,75 | 2,5 | 29,5 | 22 | 121 | 150,5 | 180/30 |
| **Pulver C** |  |  |  | 32,5 | 10 | 0 | 32,5 |  |
| **Beispiel 25** | 0,3 | 1 | 2,5 | 31 | 23,5 | 72 | 103 | 180/30 |
| **Beispiel 26** | 0,5 | 1 | 1,0 | 30 | 23 | 94 | 124 | 170/60 |
| **Beispiel 27** | 0,8 | 0,7 | 2,2 | 27 | 21 | 150 | 177 | 180/30 |
| *: 1,3-Dioxolan-2-on **: Al$_2$(SO$_4$)$_3$18H$_2$O |  |  |  |  |  |  |  |  |

**Beispiele 28 und 29:** (nicht erfindungsgemäß)

Jeweils 100 g einer pulverförmigen, vernetzten Polyacrylsäure (Pulver U), die zu 70 Mol.% als Natriumsalz vorlagen, wurden nach dem Trocknen und Mahlen auf 150 bis 180 um abgesiebt und mit Lösungen, deren Zusammensetzung in der Tabelle angegeben sind, unter kräftigem Rühren vermischt und anschließend in einem Ofen, entsprechend der unten angegebenen Bedingungen, erhitzt:

| Produkt | Al$_2$(SO$_4$)$_3$** [g] | EC* [g] | H$_2$O [g] | TB [g/g] | AAP$_{0.7}$ [g/g] | SFC [10$^{-7}$cm$^3$s/g] | TB+SFC | T/t [°C/min.] |
|---|---|---|---|---|---|---|---|---|
| **Pulver U** | | | | 31 | 11 | 0 | 30 | |
| **Beispiel 28** | 0,5 | 0,5 | 3 | 28 | 23,5 | 43 | 71 | 180/30 |
| **Beispiel 29** | 0,5 | 0,5 | 3 | 28 | 23 | 33 | 61 | 120/30 |
| **Vergleichsbeispiel 18** | | 0,5 | 3 | 28,5 | 24 | 12 | 40,5 | 180/30 |
| **Vergleichsbeispiel 19** | | 0,5 | 3 | 28 | 24 | 9 | 37 | 120/30 |
| * Ethylenglykoldiglycidylether<br>**: Al$_2$(SO$_4$)$_3$18H$_2$O | | | | | | | | |

Die Beispiele zeigen die deutliche Verbesserung der Permeabilität der erfindungsgemäßen Polymerisate im gequollenen Zustand, gekennzeichnet durch einen SFC-Wert. Die beiden anderen relevanten Parameter, die Teebeutelretention und die Flüssigkeitsaufnahme unter Druck (AAP$_{0.7}$) liegen trotz hoher Permeabilität auf hohem Niveau. Gezeigt wurde auch, daß sich nur durch die Behandlung mit einer Kombination aus organischem Nachvemetzer und einer anorganischen Salzkomponente wie Aluminiumsulfat, Aluminiumchlorid, Eisenchlorid (III), Magnesium- oder Calciumacetat mit Erhitzen des beschichteten Polymerisates eine entsprechende Eigenschaftskombination aus hohem Retentionsvermögen, guter Flüssigkeitsaufnahmefähigkeit unter Druck und hoher Permeabilität im gequollenen Zustand erreichen läßt. Nur die Verwendung der Salzkomponente (Vergleichsbeispiele 2 und 8-10) oder des organischen Nachvernetzers (Vergleichsbeispiele 1, 3 - 7 und 15 - 17) führt nicht zum gewünschten Eigenschaftsprofil. Eine nachträgliche Behandlung von bereits oberflächlich nachvernetztem Polymerisat mit einem Aluminiumsalz führt nicht zu der gewünschten deutlichen Verbesserung der Permeabilität (Vergleichsbeispiele 11 bis 14). Vielmehr ist dabei eine Schädigung der Retentions- und Flüssigkeitsaufnahmefähigkeit unter Druck zu verzeichnen. Oberflächenbehandlungen, die in den Vergleichsbeispielen beschrieben sind und aus den jeweilig angegebenen Patenten entnommen sind, führen auch nicht annähernd zu Superabsorbern, die mit den erfindungsgemäßen Produkten vergleichbar sind. Darüber hinaus treten bei der Beschichtung der Polymerisate mit großen Mengen wässriger Lösung, bzw. organischer Lösungsmittel erhebliche Probleme bezüglich der Durchführbarkeit der Verfahren auf (starkes Verklumpen des Materials, bzw. große Mengen abzuführender organischer Dämpfe).

## Patentansprüche

1.  Verfahren zur Herstellung von an der Oberfläche nachvernetzten, Wasser, wäßrige oder seröse Flüssigkeiten sowie Blut absorbierenden Polymerisaten, aufgebaut aus

    a) 55-99,9 Gew% polymerisierten, ethylenisch ungesättigten, säuregruppenenthaltenden Monomeren, die mindestens 25 Mol% neutralisiert sind,
    b) 0-40 Gew% polymerisierten, ethylenisch ungesättigten, mit a) copolymerisierbaren Monomeren,
    c) 0,1-5,0 Gew% eines oder mehrerer polymerisierter Vernetzer,
    d) 0-30 Gew% eines wasserlöslichen Polymeren
    wobei die Summe der Gewichtsmengen a) bis d) 100 Gew.% beträgt, wobei das Polymerisat mit
    e) 0,01 bis 5 Gew.%, bezogen auf das Polymerisat, eines organischen Oberflächennachvernetzungsmittels, mit Ausnahme von Polyolen, in Form einer wässrigen Lösung und mit
    f) 0,001 - 1,0 Gew%, bezogen auf das Polymerisat, eines Kations in Form einer wäßrigen Lösung beschichtet und nachvernetzt worden ist,

    **dadurch gekennzeichnet, daß** man eine Mischung aus

    a) 55-99,9 Gew% ethylenisch ungesättigten, säuregruppentragenden Monomeren, die mindestens zu 25 Mol% neutralisiert sind,
    b) 0-40 Gew% ethylenisch ungesättigten, mit a) copolymerisierbaren Monomeren,
    c) 0,1 - 5,0 Gew% eines oder mehrerer Vernetzerverbindungen,
    d) 0-30 Gew% eines wasserlöslichen Polymeren
    wobei die Summe der Komponenten a) bis d) 100 Gew.% beträgt, radikalisch polymerisiert, ggf. zerkleinert, trocknet, pulverisiert, siebt und daß man das Polymerisatpulver mit

e) 0,01 bis 5 Gew.%, bezogen auf das Polymerisat, eines organischen Oberflächennachvernetzungsmittels, mit Ausnahme von Polyolen, in Form einer wässrigen Lösung und mit

f) 0,001 - 1,0 Gew%, bezogen auf das Polymerisat, eines Kations in einer wässrigen Lösung behandelt,

wobei unter intensiven Mischen der gemeinsam oder getrennt vorliegenden, wässrigen Lösungen der Komponenten e) und f) mit dem Polymerisatpulver eine Nachvernetzung des Polymerisatpulvers erfolgt, wobei als Komponente e) 1,3-Dioxolan-2-on eingesetzt wird und wobei als Komponente f) ein Aluminium-Kation in einer wässrigen Lösung eines Aluminiumsalzes eingesetzt wird.

2. Verfahren nach Anspruch 1, **dadurch gekennzeichnet, daß** das eingesetzte Polymerisatpulver einen Feuchtigkeitsgehalt von 0,5 bis 25 Gew.% aufweist.

3. Verfahren nach Anspruch 1 oder 2, **dadurch gekennzeichnet, daß** das eingesetzte Polymerisatpulver eine Teilchengröße von < 3000 $\mu$m aufweist.

4. Verfahren nach den Ansprüchen 1 bis 3, **dadurch gekennzeichnet, daß** die wässrigen Lösungen der Komponente e) und f) vor ihrem Einsatz auf 20°C bis 100°C aufgewärmt werden.

5. Verfahren nach den Ansprüchen 1 bis 4, **dadurch gekennzeichnet, daß** die Nachvernetzung bei Temperaturen von 40°C bis 300°C erfolgt.

**Claims**

1. Process for preparing water, aqueous or serous liquids and blood absorbing polymers that are post-cured at the surface, composed of

a) 55-99.9 wt% of polymerised, ethylenically unsaturated, acid group containing monomers, which are at least 25 mol % neutralised,
b) 0-40 wt% of polymerised, ethylenically unsaturated monomers copolymerisable with a),
c) 0.1-5.0 wt% of one or more polymerised crosslinking agents,
d) 0-30 wt% of a water-soluble polymer,
wherein the sum of the amounts by weight of a) to d) comes to 100 wt%, wherein the polymer has been coated and post-cured with
e) 0.01 to 5 wt%, referred to the polymer, of an organic surface post-curing agent, with the exception of polyols, in the form of an aqueous solution and with
f) 0.001-1.0 wt %, referred to the polymer, of a cation in the form of an aqueous solution,

**characterized in that** a mixture of

a) 55-99.9 wt% of ethylenically unsaturated, acid group-bearing monomers, which are at least 25 mol % neutralised,
b) 0-40 wt% of ethylenically unsaturated monomers copolymerisable with a),
c) 0.1-5.0 wt% of one or more crosslinking compounds,
d) 0-30 wt% of a water-soluble polymer,
wherein the sum of components a) to d) comes to 100 wt %, is radically polymerised, optionally broken down, dried, pulverised, screened and that the polymer powder is treated with
e) 0.01 to 5 wt%, referred to the polymer, of an organic surface post-curing agent, with the exception of polyols, in the form of an aqueous solution and with
f) 0.001 - 1.0 wt%, referred to the polymer, of a cation in an aqueous solution,

wherein, with intensive mixing of the jointly or separately present aqueous solutions of components e) and f) with the polymer powder, a post-curing of the polymer powder takes place, wherein 1,3-dioxolan-2-one is used as component e) and wherein an aluminium cation in an aqueous solution of an aluminium salt is used as component f).

2. Process according to claim 1, **characterised in that** the polymer powder used has a humidity content of 0.5 to 25 wt %.

3. Process according to claim 1 or 2, **characterised in that** the polymer powder used has a particle size of< 3000 $\mu$m.

4. Process according to claims 1 to 3, **characterised in that** the aqueous solutions of components e) and f) are heated to 20 °C to 100 °C prior to their use.

5. Process according to claims 1 to 4, **characterised in that** the post-curing takes place at temperatures of 40 °C to 300 °C.


**Revendications**

1. Procédé de production de produit de polymérisation, réticulé en surface, absorbant l'eau, les fluides aqueux ou séreux, de même que le sang, constitué de :

   a) 55 à 99,9 % en poids de monomères polymérisés, éthyléniquement insaturés, contenant des groupes acides, qui sont au moins neutralisés à 25 % en mole,
   b) 0 à 40 % en poids de monomères polymérisés, éthyléniquement insaturés, aptes à la copolymérisation avec a),
   c) 0,1 à 5,0 % en poids d'un ou plusieurs agents réticulants polymérisés,
   d) 0 à 30 % en poids d'un polymère soluble dans l'eau,
   la somme des quantités en poids de a) à d) s'élevant à 100 % en poids, et
   le produit de polymérisation étant recouvert de
   e) 0,01 à 5 % poids, sur la base du produit de polymérisation, d'un agent organique de post-réticulation en surface, à l'exception de polyols, sous la forme d'une solution aqueuse et
   f) 0,001 à 1,0 % en poids, sur la base du produit de polymérisation, d'un cation sous la forme d'une solution aqueuse et réticulé,

   **characterisé en ce qu**'une melange de

   a) 55 à 99,9 % de monomères, éthyléniquement insaturés porteurs de groupes acides, sont neutralisés jusqu'à au moins 25 % en mole,
   b) 0 à 40 % en poids de monomères éthyléniquement insaturés, aptes à la copolymérisation avec a),
   c) 0,1 à 5,0 % en poids d'un ou plusieurs composés réticulants,
   d) 0 à 30 % en poids d'un polymère soluble dans l'eau la somme des composés a) à d) s'élevant à 100 % en poids, est polymérisée par réaction radicalaire, le cas échéant, broyée, séchée, pulvérisée, tamisée et en ce que la poudre de produit de polymérisation est traitée par
   e) 0,01 à 5 % en poids, sur la base du produit de polymérisation, d'un agent de post-réticulation de surface, à l'exception de polyols, sous la forme d'une solution aqueuse et par
   f) 0,001 à 1,0 % en poids, sur la base du produit de polymérisation, d'un cation est traitée dans une solution aqueuse,

   de façon à effectuer, par mélange intime des solutions aqueuses, présentes conjointement ou séparément des composants e) et f), avec la poudre de produit de polymérisation, une post-réticulation de la poudre de produit de polymérisation, et
   en ce qu'on utilise comme composé e) le 1,3-dioxolan-2-one et
   en ce qu'on utilise comme composé f) un cation d'aluminium dans une solution aqueuse.

2. Procédé selon la revendication 1, **caractérisé en ce que** la poudre de produit de polymérisation utilisée présente une teneur en humidité de 0,5 à 25 % en poids.

3. Procédé selon les revendications 1 ou 2, **caractérisé en ce que** la poudre de produit de polymérisation utilisée présente une granulométrie < 3 000 $\mu$m.

4. Procédé selon les revendications 1 à 3, **caractérisé en ce que** les solutions aqueuses des composés e) et f) sont chauffées, avant leur emploi, de 20°C à 100°C.

5. Procédé selon les revendications 1 à 4, **caractérisé en ce que** la post-réticulation s'effectue à des températures de 40°C à 300°C.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19646484
- US 4043952 A
- DE 4020780 A
- EP 0233067 A
- WO 9605234 A
- WO 9522356 A
- WO 9712575 A
- US 4286082 A

- DE 2706135
- US 4076663 A
- US 4340706 A
- DE 3713601
- DE 2840010
- EP 0339461 A
- DE 4020780